Europäisches Patentamt

European Patent Office

Office européen des brevets

(11) Publication number: **0 305 052**
**A1**

## (12) EUROPEAN PATENT APPLICATION

(21) Application number: 88306964.3

(22) Date of filing: 28.07.88

(51) Int. Cl.⁴: **A61L 15/01 , A61F 13/00**

(30) Priority: 30.07.87 JP 188979/87

(43) Date of publication of application:
**01.03.89 Bulletin 89/09**

(84) Designated Contracting States:
**BE CH DE FR GB IT LI SE**

(71) Applicant: **Dow Corning Kabushiki Kaisha**
**15-1, Nishishimbashi 1-chome Minato-ku**
**Tokyo 105(JP)**

(72) Inventor: **Urabe, Yosuke**
**10-14, 2-chome, Fujimino**
**Hiratsuka-shi Kanagawa Prefecture(JP)**
Inventor: **Kawashima, Hiroyuki**
**10-1, 3-chome Terao Kita**
**Ayase-shi Kanagawa Prefecture(JP)**

(74) Representative: **Laredo, Jack Joseph et al**
**Elkington and Fife Beacon House 113**
**Kingsway**
**London, WC2B 6PP(GB)**

(54) **Artificial skin and method for its production.**

(57) An improved method of making artificial skin which consists of embedding bioabsorbable fibrous material into an uncured polymeric attachment layer, which is adhered to a cured polymeric substrate film layer, and then curing the attachment layer.

EP 0 305 052 A1

Xerox Copy Centre

## Artificial Skin and Method for its Production

The present invention relates to an artificial skin which is useful as a covering material for burn wounds, external wounds, etc., suffered by humans and other animals, as well as to a method for the production of such an artificial skin.

A number of materials are known in the art as artificial skin or wound-covering materials which may be temporarily used to cover a burn wound, external wound, etc., in order to prevent the loss of body fluids, in order to prevent bacterial infection, etc., and thus promote healing. This type of artificial skin typically consists of a polymer support film and a fibrous material which is installed on the polymer support film, and it is applied and attached on the skin surface by laying it on the affected region in such a manner that the fibrous material is brought into contact with the wound surface. The use of bioabsorbable substances as the fibrous material has recently been proposed and developed.

With regard to such an artificial skin in which a bioabsorbable fibrous material is placed on the polymer support film, a number of advantages appear because the fibrous material is absorbed by the organism during wound healing, and newly formed epidermis is not damaged when the polymer support film is peeled off after the formation of new epidermis, and post-treatment is simplified.

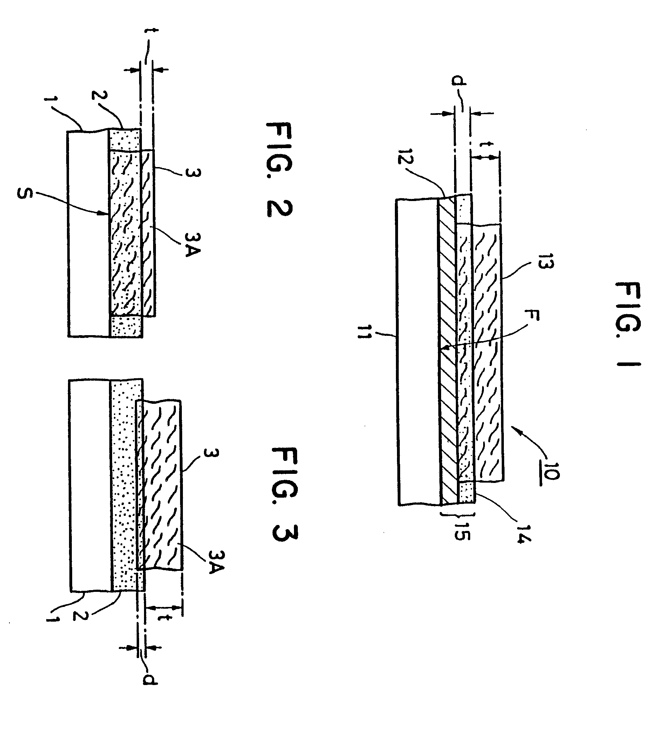

With regard to methods for the production of prior artificial skin using a bioabsorbable fibrous material, as depicted in Figures 2 and 3, polymer support film 2, on which the fibrous material 3 will be installed, is prepared by forming a layer of a support film material, for example, an uncured silicone rubber, etc., which is capable of curing to form the polymer support film 2, on the releasable surface of a releasable protective film 1 (which is peeled and removed prior to the time of application). The bioabsorbable fibrous material 3 is arranged on and press-adhered into this layer of support film material in such a manner that part of the fibrous material is embedded within the layer of support film material, and the layer of support film material is then cured in this condition.

However, bioabsorbable fibrous material 3 cannot easily be properly installed on the polymer support film 2 by methods as described above. In the step in which fibrous material 3 is press-adhered into the layer of support film material which will ultimately be cured to form the polymer support film 2, the magnitude of the force of application must be strictly regulated, even though such control is relatively difficult. As shown in Figure 2, when the force of application is excessive, the fibrous material 3 entirely penetrates the layer of support film material and is exposed on the surface S of the polymer support film 2 covered with protective film 1. Under such conditions, the artificial skin cannot exercise its expected function due to too small a thickness t of section 3A, which is the actual functional portion of fibrous material 3. Furthermore, a high strength cannot be obtained on the part of such a polymer support film 2 because the interposition of the fibrous material within polymer support film 2 causes it to become a material which is easily ruptured when stretched.

On the other hand, as shown in Figure 3, when the application pressure is too low, the fibrous material 3 is only slightly embedded in the layer of support film material, and the embedding depth d of fibrous material 3 across the thickness of polymer support film 2 will be small. In such an artificial skin, fibrous material 3 is only weakly unified with polymer support film 2, raising the possibility that the former will tear from the polymer support film 2 during its use. The value of t for section 3A, the actual functional element of fibrous material 3, is large, and a large gap is thus formed between polymer support film 2 and the treated skin surface, which raises the risk of infection. As a consequence, such a structure cannot achieve the desired functions of an artificial skin.

The present invention was developed in view of the preceding circumstances, and has as one object the introduction of an artificial skin which has a satisfactorily high strength because the bioabsorbable fibrous material is ideally installed in the polymer support film, and in which the fibrous material reliably performs its expected functions.

A second object of the present invention is to introduce a simple and reliable method for the production of artificial skin in which the bioabsorbable fibrous material is ideally installed in the polymer support film.

The artificial skin of the present invention characteristically consists of a polymer support film which itself consists of a substrate film layer and a fibrous material-attachment layer formed on said substrate film layer, and of a fibrous material of bioabsorbable substance which is fixed, in a configuration wherein only part of the fibrous material-attachment layer is embedded in the polymer support film.

With regard to the preceding, it is preferred that the fibrous material be embedded across the entire thickness of the fibrous material-attachment layer of the polymer support film, that the substrate film layer of the polymer support film be formed of a synthetic polymeric compound, and that the fibrous material-attachment layer be formed of the same type of synthetic polymeric compound as the substrate film layer.

2

An artificial skin with the above structure will always have a satisfactory flexibility and strength since the substrate film layer of the polymer support film is formed independently of the fibrous material. A satisfactory attachment strength and the reliable expected operation of the fibrous material are also obtained because the bioabsorbable fibrous material is installed in a dedicated fibrous material-attachment layer.

By embedding the fibrous material across the entire thickness of the fibrous material-attachment layer, one obtains an artificial skin having a uniformly installed fibrous material and thus obtains a very uniform artificial skin as a whole. When the substrate film layer and the fibrous material-attachment layer of the polymer support film are formed of the same type of synthetic polymeric compound, the two elements can be reliably unified and the artificial skin thus has a very strong polymer support film.

The method for the production of the artificial skin of the present invention has the characteristic that the fibrous material-attachment layer, in which the fibrous material is fixed and which together with the substrate film layer constitutes the polymer support film, is formed by press-adhering fibrous material composed of bioabsorbable substance, wherein only part of the fibrous material is embedded, into a layer of fibrous material-attachment material which has been formed on the substrate film layer and which can form the fibrous material-attachment layer by curing, and by then curing the layer of fibrous material-attachment material. It is preferred that the fibrous material be embedded across the entire thickness of the layer of fibrous material-attachment material.

When the bioabsorbable fibrous material is press-adhered by such a method into the fibrous material-attachment layer, the application pressure need not be strictly controlled in order reliably to obtain the desired embedding depth into the fibrous material-attachment layer. The production conditions are thus rendered less rigorous while an artificial skin having stable qualities and properties is easily produced at the same time.

The present invention will be explained specifically in the following with reference to the diagrams.

Figure 1 is a cross sectional view of artificial skin of the present invention.

Figure 2 is a cross sectional view of artificial skin prepared by a method of the prior art.

Figure 3 is a cross sectional view of artificial skin prepared by a method of the prior art.

Figure 1 is an explanatory cross section which shows the structure of an artificial skin 10 of the present invention. 11, 13, and 15 are, respectively, a releasable protective film, the fibrous material, and the polymer support film (composed of the substrate film layer 12 and the fibrous material-attachment layer 14). Here, the polymer support film 15, which consists of substrate film layer 12 laminated with fibrous material-attachment layer 14, is established on the releasable surface F of protective film 11, and the bioabsorbable fibrous material 13 is attached in polymer support film 15 in such a manner that a part of the bioabsorbable fibrous material 13 is embedded across the entire thickness of the fibrous material-attachment layer 14.

Such an artificial skin 10 is produced as follows according to the present invention. The releasable protective film 11 is first set up and, for example, an uncured silicone rubber is applied on the releasable surface F of the protective film 11. This is then cured by a suitable heat treatment in order to form a substrate film layer 12 consisting of a synthetic polymeric compound. A silicone rubber identical to that in the substrate film layer 12 is then applied on the substrate film layer 12 in order to form a layer of fibrous material-attachement material. Fibrous material 13, which is composed of bioabsorbable substance, is placed on and press-adhered into the layer of fibrous material-attachment material in such a way that a part of the fibrous material 13 becomes embedded across the entire thickness of the layer of fibrous material-attachment material. The layer of fibrous material-attachment material is then cured in this state by a suitable heat treatment, thus to form the polymeric fibrous material-attachment layer 14 having an attached fibrous material 13 as well as to form the polymer support film structure having a unified substrate film layer 12.

This produces an artificial skin 10 in which fibrous material 13 is mounted in the polymer support film 15 to an embedding depth d identical to the thickness of the fibrous material-attachment layer 14.

The polymer support film in the present case should (i) have a water permeability equivalent to or on the order of that of normal skin, (ii) have the flexibility necessary for the coverage of joints and other flexible body regions, (iii) have the strength necessary for attachment on the wound surface by, for example, sutures, etc., (iv) have a suitable integrity so peeling after healing may be carried out easily, (v) not be pyrogenic or antigenic, and (vi) be microbially resistant. Polymer support films which fully satisfy these requirements are preferred.

The materials comprising the substrate film layer of the polymer support film are exemplified by natural rubbers; synthetic rubbers such as silicone rubbers, urethane rubbers, fluororubbers, butadiene rubbers, styrene-butadiene rubbers, butyl rubbers, chloroprene rubbers, epichlorohydrin rubbers, etc.; polyethylene; polypropylene; polystyrene; polyvinyl chloride; polyvinyl acetate; polyvinyl alcohol; polyesters; fluororesins;

etc. Among these, silicone elastomers are preferred. Materials selected from the various silicone elastomers, such as, for example, peroxide-vulcanizing types, platinum-type catalyst-vulcanizing types, room temperature-curable types, etc., can be used. Use may also be made of silicone adhesives or the blends or copolymers of such silicones with other organic polymers.

The material constituting the fibrous material-attachment layer is not specifically restricted as long as it has good adhesion for the aforementioned substrate film layer, is soft enough (when uncured prior to the curing process) that the bioabsorbable fibrous material can be embedded by the application of pressure, and can be cured to form a strong polymeric compound which can fix the embedded segment of the fibrous material. However, preferably, the material forming the fibrous-material attachment layer is the same type as, or identical to, that in the substrate film layer, since this will reliably provide good adhesion with the substrate film layer.

The fibrous material must be integrally fixed in the fibrous material-attachment layer of the polymer support film to such a degree that the polymer support film will not separate from the treated wound surface during the healing period in which the artificial skin is applied and new epidermis grows.

The fibrous material described above is to be prepared from a bioabsorbable substance. In general, such a fibrous material is prepared by spinning a bioabsorbable polymer; however, it can be a mixed fiber of bioabsorbable polymer and nonbioabsorbable polymer. In the present sense, the bioabsorbable polymer is a substance which is degraded or modified by the physical or chemical activity of the organism's tissues when in contact with the organism's tissues and is thus absorbed by the organism. It is specifically exemplified by polyglycolic acid, polylactide, polydioxanone, polycaprolactone, glycolic acid-lactic acid copolymers (glycolide-lactide copolymers), collagen, polyamino acids, glycolide-caprolactone copolymers, amino acid-lactic acid copolymers, polylactic acid, lactic acid-caprolactone copolymers, chitin, chitosan, etc.

Specific examples of such bioabsorbable polymer fibers are, among others, the commercially available VICRYL (glycolic acid-lactic acid copolymer from Ethicon, Inc., Somerville, N.J.), PDS (polydioxanone from Ethicon, Inc.), DEXON (polyglycolic acid from Davis and Geck, Pearl River, N.Y.), etc.

The fibrous material composed of bioabsorbable material as above can be used as the monofilament, fiber tow, knit or weave, that is, as the fiber's woven or knitted fabric. A mesh-like weave or knit is particularly preferred.

The size of the fiber in the fibrous material is not specifically restricted, but generally no more than 50 denier, for example, 20 to 30 denier, is preferred. When the fibrous material is used in the form of a knit or weave, the texture of this knit or weave is not specifically restricted, and any texture may be used, such as weft-knitted fabrics such as single and double, warp-knitted fabrics such as tricot, plain weaves, twill weaves, etc., however, warp-knitted textures are optimal from such standpoints as elasticity and edge fraying. Preferably, the weight of the fibrous material is within the range of, but not limited to, 5 to 200 g/m$^2$ and, more preferably 10 to 30 g/m$^2$. Furthermore, the texture should not be completely closed, but rather a porous material with regularly arranged openings (the mesh) is preferred.

There is no specific restriction on the quantity or thickness of the fibrous material to be installed. However, the quantity (thickness) of the fibrous material, the status of the fibers which form the fibrous material, particularly the size and form of the fibers are to be appropriately selected from a consideration of the type of bioabsorbable substance actually used in the fibrous material, the condition of the wound to be treated, and other conditions.

As desired, biologically-acceptable materials can be supported on the aforesaid fibrous material composed of bioabsorbable substance. Specific examples of biologically-acceptable materials useful in this regard are collagen, gelatin, fibrinogen, albumin, other proteins, hyaluronic acid, chondroitin sulfate, other mucopolysaccharides, lysine, aspartic acid, glycine, other amino acids, lecithin, etc. Among these, the use of collagen is particularly suitable.

Such biologically-acceptable materials may be applied on fibrous material which has been subjected to a hydrolytic treatment, but the means for realizing this are not specifically restricted. Thus, the biologically-acceptable materials can be supported on the fibrous material by means appropriately selected, based on a consideration of the properties of the biologically-acceptable materials and other conditions, from methodologies such as, for example, spraying, immersion, etc. Furthermore, by applying the biologically-acceptable materials to the fibrous material followed by a chemical or heat treatment, it is possible to strengthen the bonding status by crosslinking the biologically-acceptable materials to the fibrous material.

While there is no specific restriction on the quantity of application of the biologically-acceptable materials, the amount should be enough to provide a thickness in the range of several microns to approximately 100 microns on the fibrous material. Large quantities of application may be uneconomical.

Furthermore, while it is generally sufficient to apply the biologically-acceptable materials by means such as a simple coating of the fibrous material, a primer treatment, crosslinking reaction, or other specific means of attachment may be used.

To use the artificial skin 10 with the above-described structure, it is applied by placing the surface on which fibrous material 13 is exposed in contact with the wound surface. Protective film 11 is then peeled from polymer support film 15, and the polymer support film 15 is then attached to the skin surrounding the wound using sutures, etc. The wound is thus covered with and protected by the artificial skin, and nascent epidermis grows in the wound in such a way that it also penetrates into the fibrous material. Due to this, the artificial skin under consideration has a satisfactory adhesion for the wound surface, and will not separate from the treated wound until the wound has healed. Furthermore, since the fibrous material is composed of bioabsorbable substance, it is gradually absorbed during the period of application and will be almost completely decomposed or degraded at the end of the typical healing period of 2 to 4 weeks. As a result, either the state of attachment between the fibrous material and the fibrous material-attachment layer is dissolved at the boundary of the fibrous material, or the force of attachment becomes very low. The polymer support film can then be reliably peeled by itself from the treated skin surface using very little force and without damaging newly formed epidermis.

Because the substrate film layer 12 is formed separately from the fibrous material 13 in the artificial skin of the present invention, the necessary strength and flexibility are readily obtained. Also, a satisfactory attachment strength and reliable development of the expected function are obtained for the bioabsorbable fibrous material 13 since it is fixed by a dedicated fibrous material-attachment layer 14.

Furthermore, the embedding depth d for fibrous material 13 in polymer support film 15 is quite easily made essentially equal to the thickness of the fibrous material-attachment layer 14, and attachment by fibrous material 13 in the fibrous material-attachment layer 14 can be realized in a remarkably uniform manner as a whole, which provides for the production of an artificial skin having remarkably uniform functions and properties. Also, by forming the polymer support film's 15 substrate film layer 12 and fibrous material-attachment layer 14 of the same type of synthetic polymeric compound, one obtains a secure and reliable unification of these two elements, and an artificial skin having a very strong polymer support film can be prepared as a result.

Furthermore, by way of example, the application of a biologically-acceptable materials to the fibrous material can serve, depending on the former's characteristics, to increase the compatibility (affinity) for the organism's tissue, promote the growth of new epidermis, increase adhesion for the wound surface, etc. The instant fibrous material is a quite excellent carrier for biologically-acceptable materials, and it is very easy to increase the contact area of such a biologically-acceptable materials with the wound surface by supporting it in this manner on the fibrous material. Furthermore, in some cases one can anticipate that the strength of the entire artificial skin will be increased by supporting biologically-acceptable materials on the fibrous material.

In the method of the present invention, a layer of fibrous material-attachment material is formed on the substrate film layer 12 (the latter will not be penetrated by the fibrous material 13), fibrous material 13 composed of bioabsorbable substance is press-adhered into this layer of fibrous material-attachment material, and the layer of fibrous material-attachment material is then cured to form the fibrous material-attachment layer 14 with fibrous material 13 attached. As a consequence, the fibrous material 13 is reliably and securely pressed into and embedded across the entire thickness of fibrous material-attachment layer 14 merely by setting the application pressure a little higher during press-adhesion of the fibrous material. Accordingly, the rigorous requirements on the application force are rather considerably eased, and fibrous material 13 can be reliably mounted without surface exposure occurring due to penetration across polymer support film 15 and without sacrificing the strength of polymer support film 15. The embedding depth d of the fibrous material 13 will thus always have the prescribed value, i.e., an embedding depth equal to the thickness of the fibrous material-attachment layer 14, which results in the simple and easy production of an artificial skin of stable quality and uniform properties.

The following examples are illustrative only and should not be construed as limiting the invention.

Example 1

A 40% Chlorothene dispersion of a platinum-type catalyst-vulcanizing silicone rubber, SILASTIC® Q7-4840 A/B Elastomer, available from Dow Corning Corporation, Midland, MI, was coated on the releasable surface of a releasable polyethylene terephthalate protective film in a quantity sufficient to give a thickness of 25 microns after curing. The coated dispersion was air-dried and then heated at 120 degrees Centigrade for 10 minutes in order to cure the silicone rubber and thus form the substrate film layer.

Next, a layer of fibrous material-attachment material was then formed on the substrate film layer by coating the same silicone rubber dispersion as described above in a quantity which would provide a cured thickness of 20 microns. A fibrous material, obtained by converting a polyglycolic acid fiber into a mesh-like knit, was placed on this layer of fibrous material-attachment material and was press-adhered by rolling once with a rubber roller (2 kg/4.5 cm of width) at a rate of 50 cm/minute. This was air-dried and then heated at 120 degrees Centigrade for 10 minutes in order to cure the silicone rubber constituting the layer of the fibrous material-attachment material and thus form a fibrous material-attachment layer with attached fibrous material. A 0.3% aqueous solution of atelocollagen was then coated on the fibrous material by spraying, followed by drying at room temperature for 1 day to afford an artificial skin of the present invention (Sample 1) in which the fibrous material was embedded across the entire thickness of the fibrous material-attachment layer.

## Example 2

An artificial skin with the fibrous material embedded across the entire thickness of the fibrous material-attachment layer was prepared as described in Example 1, with the exception that, in press-adhesion of the fibrous material to the layer of fibrous material-attachment material, an additional 4 kg load was applied to the same rubber roll. This was designated as Sample 2.

## Example 3

An artificial skin with the fibrous material embedded across the entire thickness of the fibrous material-attachment layer was prepared as described in Example 1, with the exception that a 30% Chlorothene dispersion of the addition-reaction curable silicone rubber, SILASTIC® Q7-4720 Elastomer, available from Dow Corning Corporation, Midland, MI, was used in place of the 40% Chlorothene dispersion of the silicone rubber, SILASTIC® Q7-4840-A/B Elastomer, used in Example 1. This was designated as Sample 3.

## Comparison Example 1

A 40% Chlorothene dispersion of the platinum-type catalyst-vulcanizing silicone rubber, SILASTIC® Q7-4840 A/B Elastomer, available from Dow Corning Corporation, Midland, MI, was applied on a protective film as described for Example 1, in a quantity which would provide a thickness of 45 microns after curing, in order to prepare a layer of a silicone rubber support film material. Fibrous material as described for Example 1 was placed on this layer of support film material and press-adhered by rolling once with a rubber roll (2 kg/4.5 cm of width) at a rate of 50 cm/minute. This was air-dried and then heated at 120 degrees Centigrade for 10 minutes in order to cure the silicone rubber comprising the layer of support film material and thus form a polymer support film. A 0.3% aqueous solution of atelocollagen was applied to the fibrous material by spraying, followed by drying at room temperature for 1 day to afford an artificial skin. This was designated as Comparison Sample 1.

This artificial skin was a material in which the fibrous material was embedded across the entire thickness of the cured silicone rubber layer and was exposed on the surface of this silicone rubber layer as well. It thus could not be introduced into practical use.

Each of the samples and the comparison sample prepared as above were subjected to tensile testing, and the results are reported in Table 1.

Table 1

| Sample | Tensile Strength (g/cm) | Elongation (%) |
|---|---|---|
| Sample 1 | 420 | 110 |
| Sample 2 | 400 | 110 |
| Sample 3 | 450 | 500 |
| Comparison Sample 1 | 280 | 65 |

As the results in Table 1 make clear, in the method of the present invention, the difference in application pressure acting on the fibrous material has very little effect on the final artificial skin product, which provides for the production of an artificial skin having a very consistent quality. Furthermore, compared with the artificial skin of Comparison Sample 1, in which a substrate film layer was not separately formed, the artificial skin of Sample 1 has both a superior tensile strength and elongation. The Sample 1 artificial skin, although equipped with a polymer support film having the same overall thickness, has a polymer support film, in accordance with the invention, which is divided into a substrate film layer located on the exterior and a fibrous material-attachment layer located on the interior, wherein the substrate film layer is formed independently and bioabsorbable fibrous material is embedded in said fibrous material-attachment layer.

## Claims

1. A method of making artificial skin comprising the steps of:
a) press-adhering an attachment layer formed of uncured polymeric material on a substrate film layer formed of cured polymeric material,
b) embedding bioabsorbable fibrous material into the uncured attachment layer so that the fibrous material is embedded substantially the entire thickness of said attachment layer and a portion of the fibrous material is exposed on the surface of said attachment layer, and
c) curing the fibrous material-embedded attachment layer while said attachment layer is in contact with said substrate film layer
whereby said attachment layer and said substrate film layer will adhere together upon curing said attachment layer.

2. A method as claimed in claim 1 wherein substantially the entire length of the fibrous material is embedded in the attachment layer.

3. A method as claimed in claim 1 wherein said press adhering step a) is before said embedding step b).

4. A method as claimed in claim 1 further comprising, before the press-adhering step a), the steps of applying a layer of uncured polymeric material on a protective film and curing the layer of uncured polymeric material to form the substrate film layer.

5. A method as claimed in claim 1 wherein said substrate film layer and said attachment layer are formed of the same type of polymeric material.

6. A method as claimed in claim 5 wherein said polymeric material is silicone.

7. An artificial skin produced by the method of any of Claims 1 to 5.

Neu eingereicht / Newly filed
Nouvellement déposé

# FIG. 1

# FIG. 2

# FIG. 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | TRANSACTIONS AMERICAN SOCIETY FOR ARTIFICIAL INTERNAL ORGANS, vol. 27, 6th-8th May 1981, pages 19-22, Anaheim, California, US; I.V. YANNAS et al.: "Prompt, long-term functional replacement of skin" * Whole article * | 1-7 | A 61 L 15/01<br>A 61 F 13/00 |
| X | US-A-3 903 882 (T. AUGURT) * Column 3, lines 15-18; column 4, line 67 - column 5, line 32; column 11, lines 1-25; claims * | 1-7 | |
| X | US-A-3 896 802 (B. WILLIAMS) * Claims; column 7, lines 25-41 * | 1-7 | |
| A | SCIENCE, vol. 215, 8th January 1982, pages 174-176, AAAS; I. YANNAS et al.: "Wound tissue can utilize a polymeric template to synthesize a functional extension of skin" * Whole article * | 1-7 | TECHNICAL FIELDS SEARCHED (Int. Cl.4)<br><br>A 61 L<br>A 61 F |

The present search report has been drawn up for all claims

| Place of search | Date of completion of the search | Examiner |
|---|---|---|
| THE HAGUE | 24-11-1988 | COUSINS-VAN STEEN G.I.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)